(19) Europäisches Patentamt
European Patent Office
Office européen des brevets

(11) **EP 4 365 158 A1**

(12) **EUROPEAN PATENT APPLICATION**

(43) Date of publication:
**08.05.2024 Bulletin 2024/19**

(21) Application number: **23205718.2**

(22) Date of filing: **25.10.2023**

(51) International Patent Classification (IPC):
***C07C 17/02*** *(2006.01)* ***C07C 17/04*** *(2006.01)*
***C07C 25/08*** *(2006.01)*

(52) Cooperative Patent Classification (CPC):
(C-Sets available)
**C07C 17/02; C07C 17/04** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.11.2022 PL 44273722**

(71) Applicant: **PCC ROKITA Spolka Akcyjna**
**56-120 Brzeg Dolny (PL)**

(72) Inventors:
- **Woloszyn, Lukasz**
  **54-438 Wroclaw (PL)**
- **Dudziak, Krzysztof**
  **63-920 Pakoslaw (PL)**
- **Wosch, Dastin**
  **46-090 Popielow (PL)**
- **Bankowski, Bartosz**
  **53-150 Wroclaw (PL)**

(54) **METHOD OF SELECTIVE PARADICHLOROBENZENE PREPARATION WITH IMPROVED CATALYTIC SYSTEM RECOVERY**

(57) Benzene and/or monochlorobenzene were chlorinated with molecular chlorine to obtain paradichlorobenzene with high selectivity. A batch reactor was used for this purpose, with a highly selective catalytic system consisting of $SbCl_3$ and a phenothiazine derivative. The entire process was improved with the introduction of a new catalytic system recovery method, which was based on returning the mother liquid containing the catalytic system to the process after prior separation from the fresh post-reaction mixture by distillation of unreacted raw materials under reduced pressure and recycling them, as well as crystallization of paradichlorobenzene from the depleted liquid after vacuum distillation. The invention has a significant effect on improving the economics of the entire process.

**Fig. 1**

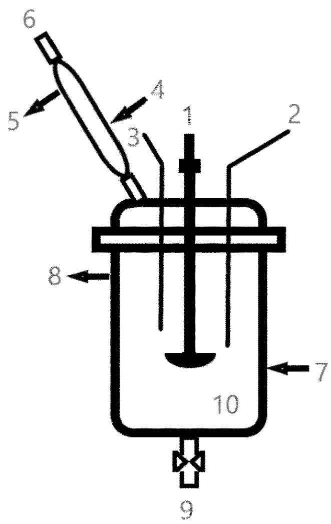


EP 4 365 158 A1

(52) Cooperative Patent Classification (CPC): (Cont.)

C-Sets
**C07C 17/02, C07C 25/08;**
**C07C 17/04, C07C 25/08**

## Description

### Field of the Invention

The present invention includes a method for selectively obtaining paradichlorobenzene with improved catalytic system recovery. Specifically, this invention includes an improved method for obtaining paradichlorobenzene by catalytically selective chlorination of a benzene/monochlorobenzene mixture. The method described herein is characterised by the recovery and return to process of a highly selective catalytic system used in paradichlorobenzene production from a benzene/monochlorobenzene mixture or from monochlorobenzene. The invention improves the efficiency and economics of the production process by reducing catalyst and cocatalyst consumption.

### Related patents and patent applications:

US2328690A; US4511552A; GB1337916A; AU251182B; GB971044A; US3354129A; WO199008181; US3029296; US2111866; US3636171; US4017551; WO2007017900A2; US3226447A; EP0126669B1; EP23551724B1; JP5375024B2; JP381832182; EP0474074A1; EP0153589B1; EP0505874A1; JP2002114719A; EP0313990A2; KR100188339B1; EP0195514B1; EP0154236B1; CN1023618418; JP565886582 (WO2010122925A1); CN109574789A; EP0118851A1; JP6287536A; EP0231133B1; EP0171265B1; CN103819307A; CN112723987A; US4300004; EP045172A; KR100352539B1; DE3617137A; DE2855940B1; US4762955A; CN102976888A

### Current state of the art

Paradichlorobenzene (PDCB) is a valuable raw material for the chemical industry. Over the years, various uses of paradichlorobenzene have been demonstrated, and the demand for this raw material continues to grow. PDCB has been used in the production of deodorants and fragrances, disinfectants, insecticides and bactericides, as well as in the formulation of pigments and dyes (e.g., US2328690A, US4511552A, GB1337916A, AU2511828, GB971044A), among others. Currently, the importance of PDCB is increasing in the context of its use in the production of polyphenylene sulfide - PPS (US3354129A), a thermoplastic plastic which, due to its high chemical and mechanical resistance, is, e.g., an alternative to metal components in the automotive industry.

Dichlorobenzenes are usually produced by chlorination of benzene and/or monochlorobenzene. Mixtures of isomers of various compositions are obtained depending on the reaction conditions and the catalyst used, containing 55-80 wt.% of para-dichlorobenzene - PDCB, 1-3 wt.% of meta-dichlorobenzene - MDCB, and 20-25 wt.% of ortho-dichlorobenzene - ODCB (Ullmann's Encyclopedia of Industrial Chemistry, ed. 5, volume A6, page 333 *et seq.*).

In the standard approach, dichlorobenzene is obtained by chlorination of benzene and/or monochlorobenzene (MCB) in the presence of a Lewis acid catalyst (Scheme 1 and 2), e.g. $FeCl_3$, $AlCl_3$, $SbCl_3$, $MgCl_2$ (WO199008181, US3029296, US2111866, US3636171, US4017551). WO2007017900A2 states that the use of the $FeCl_3$ catalyst leads to obtaining an isomeric mixture in the final product consisting mainly of 50-61% of para-dichlorobenzene (PDCB) and 30-36% of ortho-dichlorobenzene (ODCB), with a small amount *meta*-dichlorobenzene (MDCB) at 4-13%, and higher chlorinated derivatives (TCB).

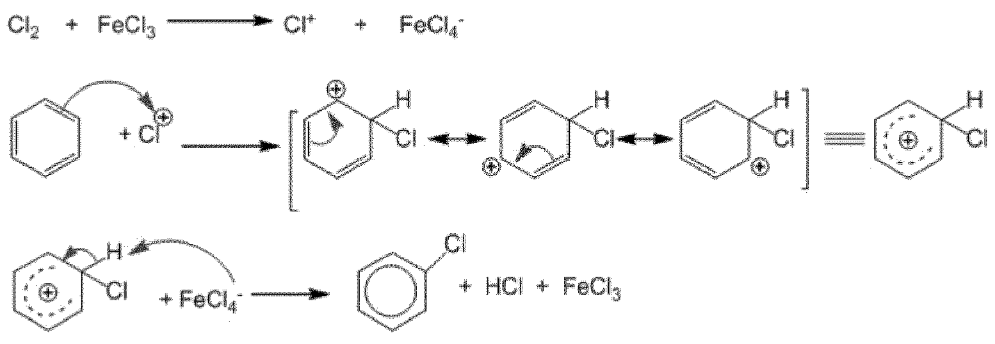


Scheme 1: Mechanism of benzene chlorination using the $FeCl_3$ catalyst (electrophilic substitution).

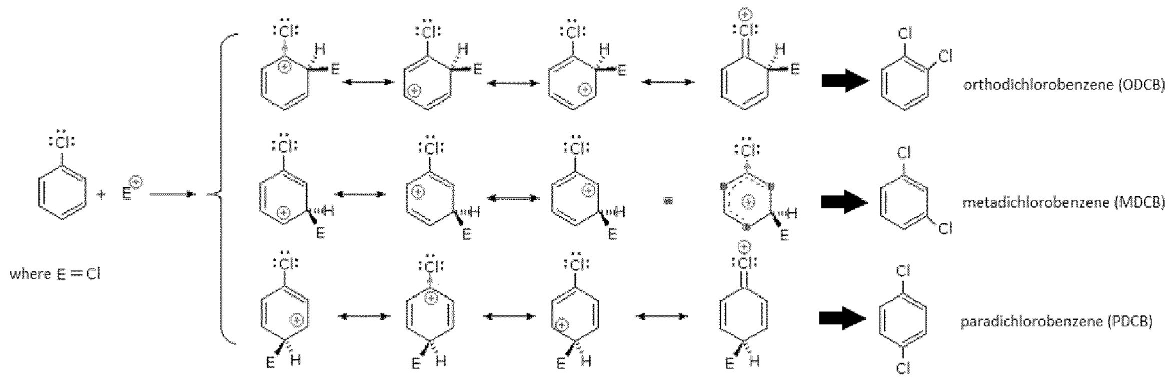


Scheme 2: Simplified mechanism of monochlorobenzene chlorination (electrophilic substitution).

From a market point of view, the PDCB isomer is the most important due to its use, e.g., in the agrochemical industry, the plastics industry and in the production of hygiene products. Exclusive use of Lewis acid-based catalysts is characterised by poor efficiency and selectivity towards PDCB. Because of that, there is an ongoing search for new catalysts or catalytic systems, the application of which would improve PDCB selectivity in relation to ODCB in the post-reaction mixture.

Lewis acid-Lewis base catalytic systems (homogeneous catalysis) or zeolites (heterogeneous catalysis) are used in order to achieve the highest possible PDCB/ODCB ratio.

WO2007017900A2 and US3226447A provide catalytic systems composed of $FeCl_3$, $AlCl_3$ (Lewis acids) and aromatic amines, sulfides, disulfides, aromatic ethers, aromatic amides, thiol derivatives (Lewis bases) that can be successfully used in the catalytic chlorination of benzene and/or MCB to PDCB. The applicants of WO2007017900A2 note that in the case of organic cocatalysts from the phenothiazine family, their price is high and the reaction requires their larger amount in order to achieve significant benzene conversion. Furthermore, a significant amount of unreacted chlorobenzene remains in the post-reaction mixture, and further reaction leads to trichlorobenzene formation as the main by-product (it is difficult to achieve satisfactory reaction efficiency with a very low trichlorobenzene level). In turn, reducing the level of the organic cocatalyst results in low benzene conversion and long reaction cycle time. Formation of detectable MDCB amounts, making the PDCB separation from the post-reaction mixture difficult, constitutes an additional problem.

EP0126669B1 and EP2351724B1 draw attention to the fact that catalytic systems with phenothiazines lead to very good selectivity for obtaining PDCB (above 80%) in chlorination process of benzene and/or monochlorobenzene. Nevertheless, the good stability of the foregoing cocatalyst allows it to be separated from the post-reaction mixture. However, the mentioned patents provide for separation by distillation, leading to the formation of heavy fractions limiting the efficiency of cocatalyst recycling.

The use of catalytic systems composed of $FeCl_3$ or $SbCl_3$ (Lewis acid) and phenothiazine derivatives (Lewis base) leads to an increase in the efficiency of the PDCB production process, and an increase in selectivity degree above 80% (EP0126669B1, EP23551724B1, JP5375024B2, JP381832182, EP0474074A1). The most data is revealed by EP23551724B1, where the molar ratio between the base and the Lewis acid in the catalytic system is specified as 0.5-1.5 (usually 1-1.5) when $FeCl_3$ was used and 0.25-2 (usually 0.5-1.5) when $SbCl_3$ was used, and the concentration of $FeCl_3$ or $SbCl_3$ is in the range of 100-2000 ppm (usually 200-1000 ppm). The benzene and chlorobenzene chlorination process described above, carried out at a temperature of 30-80°C (usually 50-70°C), allows to obtain PDCB concentration in the post-reaction mixture within the 54-58% range.

Similarly, in *Example* 2 in the patent EP0153589B1 specification, it was proven that using 10-chlorocarbonyl-10H-phenothiazine catalytic system with aluminium chloride in the benzene chlorination process at a molar ratio of 0.5:1 and at a temperature of 50°C increases the PDCB concentration in the product to approximately 58%, while maintaining selectivity *ortho-/para*-at 85%.

However, by catalysing the benzene chlorination reaction with the iron(III) chloride - benzo- and dibenzo-phenothiazine derivatives system in EP0505874A1, the PDCB concentration exceeds 50% and the selectivity is at least 82%.

On the other hand, JP2002114719A provides that it is advantageous to use the iron(III) chloride and phthalamide catalytic system when the Lewis acid concentration is 0.001 to 3 wt.%. (preferably 0.01 to 1 wt.%) based on benzene

and/or monochlorobenzene, and the molar ratio of the N-substituted phthalimide derivative to Lewis acid is 0.1-10 (preferably 0.5 to 2).

The second group of catalysts used in the industrial PDCB production process are heterogeneous catalysts - zeolites (aluminosilicates) doped with alkali metal, transition metal and rare earth metal ions, wherein their use is disclosed in patents EP0313990A2, KR100188339B1, or EP0195514B1. Moreover, the reaction on zeolite can be carried out in the presence of aliphatic carboxylic acids and their chlorides, anhydrides and salts (EP0154236B1), as well as with the addition of a cooling agent - methyl and ethyl chloride (CN102361841B). JP565886582 discloses that a catalyst containing gamma-$Al_2O_3$ as the main component can be effective in the cascade synthesis of PDCB. On the other hand, CN109574789A reports that the use of a REUSY catalyst doped with rare earth metal ions in a loop reactor shortens the PDCB synthesis time to 3-4 h.

Zeolite catalysts are characterised by better selectivity towards PDCB (e.g. EP0118851A1 reports that the use of L-type zeolite increases the selectivity of obtaining p-dichlorobenzene up to approximately 86%), but also by costs higher than Friedel-Crafts catalysts. The disadvantage is the generally high requirement for the amount of zeolite in relation to the substrate (2-6 wt.%), as well as the fact that the chlorination waste gas produced in this case generally contains, significant amounts of unused chlorine in addition to hydrogen chloride (EP0474074A1). According to JP5375024B2, the selectivity of PDCB reaches 87.6% in the technology of catalyzing the PDCB synthesis reaction using zeolites, however, long-term use of zeolite reduces the chlorine conversion level from 99.3% to approximately 80%, as caused by the production of polychlorine products that block zeolite pores and reduce its catalytic activity, which is also confirmed by the state of the art disclosed in JP6287536A. It is necessary to purify and filter the product after the reaction, especially when the zeolite is used in powder form. Therefore, according to EP0231133B1, it is preferable if the catalyst is formed into an appropriate shape and size, such as a spherical or columnar pellet or granule, and e.g. a fixed bed, suspended bed or fluidised bed is used. Zeolite powder does not clump and has poor formability. Therefore, an inorganic compound other than zeolite, e.g., alumina or silica-alumina, is often used as the binder for forming the zeolite powder. Nevertheless, binder added to improve formability sometimes has a detrimental effect on the catalytic reaction, and therefore when a binder-containing catalyst is used, it is often difficult to obtain the same activity and selectivity as with powdered zeolite. When the $Al_2O_3$ content in the aluminosilicate is 0.01-30%, according to EP0171265B1, the selectivity of the reaction towards PDCB quickly decreases. In this context, however, a better solution is to use homogeneous catalysts, e.g. cocatalytic systems containing a phenothiazine derivative (EP2351724B1), which, even when reused, allow maintaining a very high degree of selectivity (at least 83%) and almost complete chlorine conversion (over 99%).

According to the state of the art disclosed in CN103819307A, WO2007017900A2, JP565886582 (WO2010122925A1) and CN112723987A, the catalytic process for the selective chlorination of benzene and/or monochlorobenzene can be effectively carried out in a batch reactor; columnar and tubular or in their cascade; loop and microchannel reactor. The loop reactor provides better mixing and promotes reactant diffusion, and also as improves heat transfer. On the other hand, using a microchannel reactor enables easier process automation.

The chlorination process can be carried out periodically or continuously. CN112723987A indicates that batch and continuous processes are not devoid of drawbacks. According to the authors of the foregoing patent, in the case of a batch process, this includes high raw material consumption, poor heat transfer effect, low efficiency and low safety level. However, in the case of a continuous process, it is poor efficiency of mass and heat transfer and the inability to precisely control the reaction rate, which results in complex components of the obtained chlorinated liquid, and therefore difficult separation and increased production costs. Therefore, it is particularly important that the chlorination process proceeds with the highest possible mass and heat transfer and that the composition of the post-reaction mixture is optimally controlled.

As a result of chlorination of benzene and/or monochlorobenzene in the product, an isomeric mixture of dichloroderivatives is obtained, which must be separated and purified. The composition of the isomeric mixture depends largely on the type of catalyst and the chlorination reaction conditions. US4300004 discloses a five-step separation process. In the first one, a mixture of PDCB and MDCB is distilled, which have similar boiling points (172-174°C), and ODCB remains in the reactor (boiling point: 180°C). Next, the distillate is cooled first to a temperature of 10-40°C, thanks to which PDCB can be separated in the solid phase, and then further from -40°C to -5°C, which results in second PDCB solid phase separation from the liquid. In the next stages, the remaining post-crystallization liquid is distilled to remove traces of MDCB, obtaining an isomeric mixture in the distillate. Since the difference in the boiling points of each isomer is very small, the conventional distillation method only allows the mixture to be separated into two parts: ODCB (bottom product) and PDCB/MDCB (distillate).

As previously emphasised, this method of separating dichlorobenzene isomers results in the formation of heavy fractions, thus reducing the efficiency of the process and preventing effective recycling of the expensive catalyst.

The inventors of EP045172A propose extractive distillation. This method often requires expensive extractants, e.g. alkylene carbonate, and the separation of MDCB and PDCB is not satisfactory. On the other hand, sulfonation or bromination of the PDCB/ODCB mixture can very easily convert MDCB into sulfo- or bromo-DCB, which facilitates separation from unreacted PDCB. This method is disadvantageous in that desulfonation or debromination must be

carried out continuously to recover MDCB (KR100352539B1, DE3617137A).

Crystallization and distillation can be combined (DE2855940B1) to increase the MDCB content up to a eutectic mixture (approx. 85 wt.% of MDCB/approx. 15 wt.% of PDCB). However, distillation in this case is energy-intensive and time-consuming.

According to US4762955A, by cooling the reaction product to a temperature of -20° to 10°C, most of the PDCB contained therein precipitates as crystals, and by filtering the crystalline suspension thus obtained, most of the PDCB is removed to obtain a crude ODCB consisting essentially of 60-85 wt.% ODCB, 15-40 wt.% PDCB, and 0.02-2 wt.% MDCB. Then, after distilling the PDCB and MDCB isomers from the obtained raw ODCB, purified ODCB is obtained, essentially with a composition of 80-99 wt.% ODCB, 0.1-20 wt.% PDCB, and 0-1 wt.% MDCB.

CN102976888A discloses the possibility of separating PDCB from the reaction mixture by continuous crystallization carried out in a system of crystallization kettles connected in series.

**Problem in the State of the art**

In industrial practice, in order to improve the selectivity of the benzene and/or monochlorobenzene chlorination process using chlorine gas, a catalytic system is used consisting of a catalyst (Lewis acid, e.g. $FeCl_3$, $SbCl_3$, $AlCl_3$) and a cocatalyst (organic or inorganic ligand, e.g., phenothiazine and its derivatives, phthalate derivatives). Despite their high catalytic capacity, the organic cocatalysts used are usually expensive and not readily available on the market, making their use in large-scale industrial processes uneconomical. Moreover, due to the high boiling points and densities of the organic cocatalysts used, during the separation of reaction products, these substances pass to heavy fractions, treated as waste, making their recovery and recirculation to the process impossible.

Having considered the foregoing, improving the catalytic chlorination of benzene and/or monochlorobenzene with an effective and efficient catalyst recovery method is a key element to improve the economics of the entire process.

A production process for the preparation of para-dichlorobenzene by chlorination of benzene and/or monochlorobenzene (MCB) with chlorine molecules in the liquid phase, catalyzed by a Lewis acid (iron, aluminium, antimony or sulfur halide) and an organic cocatalyst based on phenothiazine analogues has been disclosed. However, the fundamental problem in the above-mentioned process, it is necessary to maintain high selectivity in obtaining para-dichlorobenzene while reducing the consumption of homogeneous catalyst/cocatalyst through its recovery in further stages of the process.

According to the state of the art disclosed in EP2351724B1, the antimony trichloride and/or iron(III) chloride and phenothiazine analogue catalytic system used in selective chlorination can be recovered in whole or in part from the distillation residue stream, and the necessary rest should be supplemented with fresh catalyst. According to the authors, distillation is carried out at atmospheric pressure or under reduced pressure, ordinary continuous or periodic, simple or multi-stage distillation is used (the ratio of the catalyst and cocatalyst concentrations during distillation is optional, it ranges from 10 to 300 times). The disadvantage of this solution is the fact that when the recovered catalytic system concentration is insufficient, recycling is carried out when a large amount of the desired product para-dichlorobenzene is found in the depleted liquid residue, which deteriorates the efficiency of the entire production.

The information disclosed in the foregoing patent application on the possibility of recovery and return of the catalytic system to the process is rather general, it does not exhaust the description of the potential stages of recovery and parameters related to it, and besides, the authors focus only on high-temperature treatment of the post-reaction liquid, which is fraught with high energy consumption, and heavy fractions (polymerization) or even weight loss of the catalyst itself may appear in the recycle liquid containing the catalyst.

In the chlorination process of benzene and/or monochlorobenzene (MCB) carried out under the described invention, despite a significant increase in the PDCB share in the stream after the reaction and maintaining high selectivity of PDCB to ODCB, the use of a homogeneous N-chlorocarbonylphenothiazine cocatalyst is economically ineffective. The high cost and the problem of organic N-chlorocarbonylphenothiazine cocatalyst used availability mean that only an effective and efficient method of its recovering, returning to the reactor and reusing in the production process will improve the profitability of the entire production.

Surprisingly, it turned out that the catalyst system used in chlorination can be effectively recovered and returned to the process, while still being able to highly selectively catalyze the PDCB production process.

**Object of the invention**

Therefore, the aim of the present invention is a method for obtaining paradichlorobenzene in the process of catalytically selective chlorination of a benzene/monochlorobenzene mixture, improved by catalytic system recovery and its return to the process for reuse.

**Disclosure of the essence of the invention**

According to the present invention, the subject of the invention is a method for selective preparation of paradichlorobenzene with improved recovery of the catalytic system, comprising the chlorination of benzene and/or monochlorobenzene with molecular chlorine in the presence of a catalytic system comprising antimony trichloride as a catalyst and N-chlorocarbonylphenothiazine as an organic cocatalyst, characterised in that

(a) the reaction mixture consisting of fresh raw material input and streams obtained as a result of recycling the fraction of unreacted raw materials together with recovery of the catalytic system is subjected to chlorination, and then

(b) the catalytic system is recovered by separating the fresh post-reaction mixture obtained in (a) in the following stages:

(I) distilling off light fractions under reduced pressure from the fresh post-reaction mixture, including unreacted benzene and/or monochlorobenzene;

(II) crystallization of the desired paradichlorobenzene with the collection of paradichlorobenzene as a pure product stream; after which

(III) the obtained mother liquor containing the catalytic system and the fraction of unreacted benzene and/or monochlorobenzene raw materials from (I) is recycled.

Preferably, in step (a) of this method, a reaction mixture is provided, consisting of a uniform mixture of fresh raw material input containing benzene and/or monochlorobenzene and a fraction of unreacted raw materials together with the recovered catalytic system, delivered homogeneously directly to the reactor.

Preferably, in step (a) of this method, the catalytic system is used in the amount of: 0.1 wt.% antimony trichloride relative to the total weight of the reaction mixture.

Preferably, a reaction mixture containing benzene and/or monochlorobenzene in a volume ratio of 0-75 vol% of benzene and 25-100 vol% of monochlorobenzene, more preferably 0-25 vol% of benzene and 75-100 vol% of monochlorobenzene is used in step (a) of this method.

Preferably, the chlorination process is carried out in the temperature range from 50 to 70°C, more preferably at 60°C, in step (a) of this method.

Preferably, N-chlorocarbonylphenothiazine is used with antimony trichloride at a molar ratio of 0.5-1.5:1, preferably at a molar ratio of 1:1, in step (a) of this method.

Preferably, the catalytic system is supplemented depending on the amount of recovered antimony trichloride and N-chlorocarbonylphenothiazine in step (a) of this method.

Preferably, in step (a) of this method, the mother liquor from (III) containing the catalytic system is used as recovery for recycle, more preferably in amounts above 55% of the catalyst and above 60% of the cocatalyst, based on the input amount.

Preferably, in step (a), the reaction mixture is supplemented with a fresh portion of raw materials consisting of benzene and/or monochlorobenzene, and optionally a fresh portion of the catalytic system for recovery from step (b).

Preferably, in step (a), chlorine gas is introduced into the reactor at a pressure of 0.1-5 bar, more preferably 0.3-1 bar, and most preferably 0.4-0.5 bar.

Preferably, in step (a) of this method, the chlorination process is carried out for 4-15 h, preferably 6-10 h.

Preferably, a three-step separation process is used in step (b) of this method, comprising vacuum distillation, crystallization, most preferably melt crystallization, and filtration, while maintaining the highly selective catalytic capacity of the above-mentioned catalytic system.

Preferably, the distillation step (I) for benzene is carried out under vacuum conditions of 0-1000 mbar, more preferably 120-180 mbar, and most preferably 140-145 mbar - resulting in a boiling point of 45-50°C and/or for monochlorobenzene it is carried out at vacuum conditions 0-1000 mbar; more preferably 60-100 mbar, and most preferably 80-90 mbar - resulting in a boiling point of 55-60°C.

Preferably, step (II) of this method involves crystallization of the desired paradichlorobenzene with collection of paradichlorobenzene as a stream of pure product in crystalline form or in melt form.

Preferably, crystallization is used in step (II) of this method, involving slowly lowering the temperature at a rate of 0.1-5°C/min, preferably 0.1-1°C/min; and most preferably 0.2-0.3°C/min, while mixing slowly in the range of 20-100 rpm, and most preferably 50-60 rpm.

Preferably, in step (III) of this method, the mother liquor obtained by filtration is recycled into (a).

Preferably, the method according to the invention is carried out in a batch reactor, a continuous reactor or in

a tubular reactor, a microchannel reactor, an overflow reactor with a mechanical stirrer, a "bubble column" reactor, a "loop-reactor" or any other types of reactors.

Advantageously, in step (a) of this process, another Lewis acid including iron chloride, aluminium chloride, magnesium chloride and zinc chloride, preferably iron chloride or aluminium chloride, may be used instead of antimony trichloride.

## Benefits and effects of the invention

The use of an efficient recovery method and recycling of the used catalytic system, i.e. catalyst (antimony trichloride) and cocatalyst (N-chlorocarbonylphenothiazine) through a multi-stage separation process including distillation under reduced pressure, crystallization and filtration, while maintaining the highly selective catalytic capacity of the foregoing catalytic system is economically effective.

## Detailed description of the invention

The present invention is illustrated in the following embodiments using a reactor and an absorber, where

**FIG. 1** shows a reactor used according to the method of the invention, wherein

1 - chlorine/nitrogen dosing line connected to a bubbling head (PTFE tube with a diameter of 6 mm)
2 - thermocouple
3 - sampler
4 - heating medium input from the thermostat (water)
5 - heating medium output to the thermostat (water)
6 - HCl and possibly $Cl_2$ waste gas line - outlet (PTFE tube dia. 12 mm)
7 - heating medium input from the thermostat (silicone oil)
8 - heating medium output to the thermostat (silicone oil)
9 - bottom drain connector
10 - glass reactor with a dome, capacity: 2 L.

**FIG. 2** shows an absorber used according to the method of the invention, wherein

11 - glass flask with a capacity of 1-6 L (depending on the length of the experiment)
12 - absorber column filled with silica gel
13 - absorber cooler
14 - sampler
15 - HCl waste gas line, possibly $Cl_2$ waste gas line - input (PTFE tube, dia. 12 mm)
16 - diaphragm pump
17 - cooling medium input to the system: absorption column + absorber cooling tower from the thermostat (water)
18 - cooling medium outlet from the absorption column + absorber cooling system to the thermostat (water)
19 - residual waste gas line for ventilation - outlet (PTFE tube, dia. 12 mm).

## Exemplary Embodiments of the Invention

The following raw materials were used in accordance with the invention:

- chlorine: purity - min. 99.8% (chlorine N28); origin - Air Liquide
- benzene: purity - min. 99.9%; origin - Chlorobenzene Production Department, Chlorine Complex, PCC Rokita SA
- chlorobenzene: purity - min. 99.9%; origin - Chlorobenzene Production Department, Chlorine Complex, PCC Rokita SA
- antimony trichloride: purity - p.a.; origin - Chempur
- N-chlorocarbonylphenothiazine: purity - min. 97%; origin - Finetech Industry Ltd. (China)
- sodium lye: conc. 50%, origin - Chlorine Production Department, Chlorine Complex, PCC Rokita SA

**General embodiment**

According to the invention, a reaction mixture containing benzene and monochlorobenzene with a volume of 1400 mL (1.4 L) was prepared in a ratio of 0-75% vol. benzene and 25-100% vol. monochlorobenzene (most preferably 0-25% vol. benzene and 75-100% vol. monochlorobenzene).

A catalytic system was introduced into the reaction mixture: antimony trichloride (CAS No. 10025-91-9) - an inorganic catalyst, the so-called Lewis acid and N-chlorocarbonylphenothiazine (CAS No. 18956-87-1) - organic cocatalyst (Scheme 3).

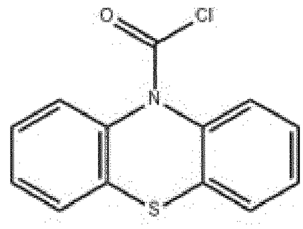

Scheme 3: Organic cocatalyst: N-chlorocarbonylphenothiazine

The catalytic system was introduced in the following amounts: 0.1 wt.% of $SbCl_3$ relative to the weight of the reaction mixture and N-chlorocarbonylphenothiazine at a molar ratio of 0.5-1.5:1 mol of Lewis acid (most preferably an organic cocatalyst in a molar ratio of 1:1 mol of Lewis acid).

The reaction mixture, together with the dissolved catalytic system, after intensive mixing with a magnetic stirrer for approx. 20 min, is introduced into the batch reactor (FIG. 1, No. 10) and is bubbled with nitrogen introduced into the reactor using a Teflon "head" (FIG. 1, No. 1) for 30-45 min, with a nitrogen flow of 30 L/h and a pressure of 0.5 bar, in order to remove trace amounts of moisture and atmospheric oxygen from the liquid introduced to the reactor, and to achieve better mixing. The reactor is thermostated (FIG. 1, no. 7-8) at 60°C - process temperature. Next, chlorine gas was introduced (purity min. 99.8%) at a pressure of 0.1-5 bar (preferably 0.3-1 bar; most preferably 0.4-0.5 bar) and a flow of 8-25 L/h (most preferably: 10-12.5 L/h) . The chlorination process was carried out for 4-15 h, preferably 6-10 h.

During the process, hydrogen chloride gas is released as a by-product, which is released through the cooler (FIG. 1, No. 6) and a line made of teflon (FIG. 2, No. 15), goes to the absorber (FIG. 2, No. 11) with a capacity of 1-6 l (depending on the process being carried out), filled with sodium lye with a concentration of 10-20% (depending on the process being conducted) for neutralization. The absorber has an internal lye circulation in which the lye moves at a flow of 5.50 L/h (FIG. 2, no. 16).

The colourless reaction mixture of benzene and monochlorobenzene takes on a greenish tint after the addition of the catalytic system. After the first portions of chlorine are introduced, the colour of the mixture changes to brown, and then from brown to black, depending on the amount of chlorine dosed (chlorination time). This is most likely related to the formation of an active catalytic complex between $SbCl_3$ and N-chlorocarbonylphenothiazine, which is initiated by contact of the above-mentioned substance with chlorine.

The selectivity of the catalyst in the experiments performed was calculated on the basis of the final percentage values of paradichlorobenzene and orthodichlorobenzene according to the formulas:

a/

***(paradichlorobenzene*100%)/(paradichlorobenzene+orthodichlorobenzene),***

and

b/***paradichlorobenzene/ortodichlorobenzene.***

The post-reaction mixture contains: benzene and/or monochlorobenzene (unreacted substrates); paradichlorobenzene and orthodichlorobenzene (main products); catalytic system "antimony trichloride-N-chlorocarbonylphenothiazine" and possibly trace amounts of metadichlorobenzene and trichlorobenzenes. An efficient method of recovering the mentioned catalytic system from the post-reaction mixture was developed according to the invention. This method includes three steps:

I/ Distillation under reduced pressure from the post-reaction mixture of light fractions of unreacted substrates, i.e. benzene (vacuum 0-1000 mbar; preferably 120-180 mbar; most preferably 140-145 mbar - which gives a boiling

point of 45-50°C) and/or monochlorobenzne (vacuum 0-1000 mbar; preferably 60-100 mbar; most preferably 80-90 mbar - which gives a boiling point of 55-60°C).

II/ Crystallization of paradichlorobenzene from the post-reaction liquid and collecting the pure product in crystalline or melt form.

III/ Recycling for rechlorination: (i) mother liquor obtained at the crystallization stage, which contains the catalytic system, and (ii) unreacted substrates - benzene and/or monochlorobenzene, recovered in the first stage, as well as supplementing the process system with a fresh portion of raw materials (benzene and/or monochlorobenzene) and possibly supplementing catalyst losses.

The recovered and recycled catalyst system [in amounts above 55% of the catalyst and above 60% of the cocatalyst in relation to the input amount] is still able to highly selectively catalyze the process of obtaining PDCB.

**Example 1 (according to the invention)**

0.1% $SbCl_3$ and N-chlorocarbonylphenothiazine at a 1/1 mol ratio with antimony trichloride were introduced into 1400 mL of the benzene/monochlorobenzene mixture (in a volume ratio of 75%/25%). After intensive mixing for about 20 minutes, the liquid turned azure (celadon) and remained clear. After introducing the process liquid into the reactor and obtaining a temperature of 60°C, chlorine was dosed at a rate of 12.5 L/h, under a pressure of 0.5 bar. Chlorination was carried out for 6 h. In contact with chlorine, the process mixture changed colour from azure to burgundy (to black), while remaining clear.

The following was obtained: benzene 49.6 wt.%; monochlorobenzene 47.1 wt.%; paradichlorobenzene 2.6 wt.%; metadichlorobenzene <0.1 wt.%; orthodichlorobenzene 0.6 wt.%

Calculated paradichlorobenzene/orthodichlorobenzene selectivity: 81.25% (4.33).

**Example 2 (according to the invention)**

0.1% $SbCl_3$ and N-chlorocarbonylphenothiazine at a 1/1 mol ratio with antimony trichloride were introduced into 1400 mL of the benzene/monochlorobenzene mixture (in a volume ratio of 25%/75%). After intensive mixing for about 20 minutes, the liquid turned azure (celadon) and remained clear. After introducing the process liquid into the reactor and obtaining a temperature of 60°C, chlorine was dosed at a rate of 12.5 L/h, under a pressure of 0.5 bar. Chlorination was carried out for 6 h. In contact with chlorine, the process mixture changed colour from azure to burgundy (to black), while remaining clear.

The following was obtained: benzene 8.9 wt.%; monochlorobenzene 79.5 wt.%; paradichlorobenzene 9.4 wt.%; metadichlorobenzene <0.1 wt.%; orthodichlorobenzene 2.0 wt.%

Calculated paradichlorobenzene/orthodichlorobenzene selectivity: 82.46% (4.70).

**Example 3 (according to the invention)**

Conditions as in Example 2, except that the chlorine dosing rate was increased to 25 L/h.

The following was obtained: benzene 3.9 wt.%; monochlorobenzene 73.4 wt.%; paradichlorobenzene 18.5 wt.%; metadichlorobenzene <0.1 wt.%; orthodichlorobenzene 3.9 wt.%

Calculated paradichlorobenzene/orthodichlorobenzene selectivity: 82.59% (4.74).

**Example 4 (according to the invention)**

Conditions as in Example 2, except that N-chlorocarbonylphenothiazine was introduced in a ratio of 1.5/1 mol with antimony trichloride.

The following was obtained: benzene 8.1 wt.%; monochlorobenzene 79.4 wt.%; paradichlorobenzene 10.1 wt.%; metadichlorobenzene <0.1 wt.%; orthodichlorobenzene 2.2 wt.%

Calculated paradichlorobenzene/orthodichlorobenzene selectivity: 82.11% (4.59).

**Example 5 (according to the invention)**

Conditions as in Example 2, except that N-chlorocarbonylphenothiazine was introduced in a ratio of 0.5/1 mol with antimony trichloride.

The following was obtained: benzene 9.0 wt.%; monochlorobenzene 79.8 wt.%; paradichlorobenzene 9.0 wt.%;

metadichlorobenzene <0.1 wt.%; orthodichlorobenzene 2.0 wt.%

Calculated paradichlorobenzene/orthodichlorobenzene selectivity: 81.82% (4.50).

**Example 6 (according to the invention)**

0.1% $SbCl_3$ and N-chlorocarbonylphenothiazine at a 1/1 mol ratio with antimony trichloride were introduced into 1400 mL of monochlorobenzene. After intensive mixing for about 20 minutes, the liquid turned azure (celadon) and remained clear. After introducing the process liquid into the reactor and obtaining a temperature of 60°C, chlorine was dosed at a rate of 12.5 L/h, under a pressure of 0.5 bar. Chlorination was carried out for 6 h. In contact with chlorine, the process mixture changed colour from azure to burgundy (to black), while remaining clear.

The following was obtained: monochlorobenzene 75.4 wt.%; paradichlorobenzene 20.2 wt.%; metadichlorobenzene <0.1 wt.%; orthodichlorobenzene 4.2 wt.%

Calculated paradichlorobenzene/orthodichlorobenzene selectivity: 82.79% (4.81).

**Example 7 (according to the invention)**

Conditions as in Example 6, except that the chlorine dosing rate was increased to 25 L/h.

The following was obtained: monochlorobenzene 68.5 wt.%; paradichlorobenzene 25.9 wt.%; metadichlorobenzene <0.1 wt.%; orthodichlorobenzene 5.4 wt.%

Calculated paradichlorobenzene/orthodichlorobenzene selectivity: 82.75% (4.80).

**Example 8 (according to the invention)**

Conditions as in Example 6, except that N-chlorocarbonylphenothiazine was introduced in a ratio of 1.5/1 mol with antimony trichloride.

The following was obtained: monochlorobenzene 74.1 wt.%; paradichlorobenzene 21.3 wt.%; metadichlorobenzene <0.1 wt.%; orthodichlorobenzene 4.4 wt.%

Calculated paradichlorobenzene/orthodichlorobenzene selectivity: 82.88% (4.84).

**Example 9 (according to the invention)**

Conditions as in Example 6, except that N-chlorocarbonylphenothiazine was introduced in a ratio of 0.5/1 mol with antimony trichloride.

The following was obtained: monochlorobenzene 75.9 wt.%; paradichlorobenzene 19.7 wt.%; metadichlorobenzene <0.1 wt.%; orthodichlorobenzene 4.1 wt.%

Calculated paradichlorobenzene/orthodichlorobenzene selectivity: 82.77% (4.80).

**Example 10 (according to the invention)**

Conditions were the same as in Example 2, except that the chlorination time was extended to 10 h.

The following was obtained: benzene 6.2 wt.%; monochlorobenzene 76.9 wt.%; paradichlorobenzene 13.8 wt.%; metadichlorobenzene <0.1 wt.%; orthodichlorobenzene 2.9 wt.%; trichlorobenzenes <0.1 wt.%; N-chlorocarbonylphenothiazine 900 ppm; antimony 520 ppm.

Calculated paradichlorobenzene/orthodichlorobenzene selectivity: 82.63% (4.76).

**Example 11 (according to the invention)**

Conditions were the same as in Example 6, except that the chlorination time was extended to 9 h.

The following was obtained: monochlorobenzene 68.1 wt.%; paradichlorobenzene 26.3 wt.%; metadichlorobenzene 0.1 wt.%; orthodichlorobenzene 5.5 wt.%; trichlorobenzenes 0.1 wt.%; N-chlorocarbonylphenothiazine 933 ppm; antimony 550 ppm.

Calculated paradichlorobenzene/orthodichlorobenzene selectivity: 82.70% (4.78).

**Example 12a (according to the invention)**

Using half the volume of the post-reaction liquid from Example 11, the catalytic system was recovered according to the method of the present invention. For this purpose, a fraction of unreacted monochlorobenzene was distilled under reduced pressure at a pressure of 80-90 mbar and a boiling point of 55-60°C, and the depleted liquid was transferred

to a cryostat, where the temperature was lowered at a rate of 0.2°C/min in the range of 20° C to -10°C, while stirring with a mechanical mixer at a speed of 50-60 rpm. The PDCB crystalline phase obtained in this way was separated from the mother liquid using a pressure filter with a length of 20 cm and diameter of 4.5 cm The mother liquor containing the recovered catalytic system was used in rechlorination (Example 12a).

The catalyst recovery rate was calculated from the formula:

***(mass of recovered catalyst*100%)/initial mass of catalyst***

The calculated recovery rate of antimony trichloride is 58%.

The calculated recovery rate of N-chlorocarbonylphenothiazine is 71%.

The organic cocatalyst is partially chlorinated during the process, therefore, in order to calculate the recovery rate, its mass was converted to the mass of a non-chlorinated molecule.

**Example 12b (according to the invention)**

The mother liquor (158 mL) and unreacted monochlorobenzene (386 mL) from Example 12a were returned to the process and supplemented with an appropriate fresh portion of monochlorobenzene (856 mL) and the catalyst system (catalyst 0.3042 g; cocatalyst 0.2410 g), followed by chlorination under conditions as in Example 11.

The following was obtained: monochlorobenzene 54.7 wt.%; paradichlorobenzene 36.3 wt.%; metadichlorobenzene 0.1 wt.%; orthodichlorobenzene 8.2 wt.%; trichlorobenzenes 0.1 wt.%; N-chlorocarbonylphenothiazine 940 ppm; antimony 510 ppm.

Calculated paradichlorobenzene/orthodichlorobenzene selectivity: 81.57% (4.43).

**Example 13a (reference)**

Using half the volume of the post-reaction liquid from Example 11, the catalytic system was recovered by distillation under reduced pressure according to EP2351724B1*,* separating the fraction of unreacted monochlorobenzene and the fraction of paradichlorobenzene that solidified in the receiver from the post-reaction liquid. The obtained depleted liquid contained the catalytic system and orthodichlorobenzene. Heavy fraction concentration (TCB): 1 wt.%.

The calculated recovery rate of antimony trichloride is 22%.

The calculated recovery rate of N-chlorocarbonylphenothiazine is 60%.

The organic cocatalyst is partially chlorinated during the process, therefore, in order to calculate the recovery rate, its mass was converted to the mass of a non-chlorinated molecule.

**Example 13b (reference)**

The depleted liquid (10 mL) and unreacted monochlorobenzene (447 mL) from Example 13a were returned to the process and supplemented with an appropriate fresh portion of monochlorobenzene (943 mL) and the catalyst system (catalyst: 0.5649 g; co-catalyst: 0.3324 g), and then chlorination was carried out under conditions as in Example 11.

The following was obtained: monochlorobenzene 53.4 wt.%; paradichlorobenzene 38.6 wt.%; metadichlorobenzene 0.1 wt.%; orthodichlorobenzene 7.9 wt.%; trichlorobenzenes 0.1 wt.%; N-chlorocarbonylphenothiazine 903 ppm; antimony 535 ppm.

Calculated paradichlorobenzene/orthodichlorobenzene selectivity: 83.01% (4.88).

**Discussion of results**

Documents from the prior art (EP0126669B1 and EP2351724B1) draw attention to the fact that catalytic systems with phenothiazines lead to very good selectivity for obtaining PDCB (above 80%) in the chlorination process of benzene and/or monochlorobenzene. Nevertheless, the good stability of the foregoing cocatalyst allows it to be separated from the post-reaction mixture. However, the aforementioned patents (EP0126669B1 and EP2351724B1) provide for separation by deep distillation, which, according to Example 13a of the present invention, generates the formation of organochlorine derivatives in the form of heavy fractions, limiting the efficiency of cocatalyst recycling.

An additional problem known from the state of the art, e.g. WO2007017900A2, is the formation of a detectable amount of MDCB, making the separation of PDCB from the post-reaction mixture difficult, which is practically eliminated according to the present invention (MDCB is absent or its concentration does not exceed 0.1%).

The invention presented herein provides for the recovery, return to the reactor and reuse of the catalyst system in the production process, using an optimized sequence of unit processes, namely:

(I) vacuum distillation from the fresh post-reaction mixture of light fractions (benzene, chlorobenzene), and then
(II) crystallization of the product (preferably by melt crystallization) and
(III) recycling of the mother liquor containing the catalyst with the co-catalyst and possibly unreacted benzene and/or monochlorobenzene.

This method of conducting the process significantly reduces energy consumption, but first and foremost: it inhibits the undesirable processes of formation of heavy fractions during deep distillation of the post-reaction mixture. The possibility of recycling the catalyst in the form of a low-viscosity liquid that easily mixes with the fresh feedstock is an additional advantage. All the foregoing features translate significantly into the efficiency and economics of the process.

Moreover, using melt crystallization in step (ii) is very beneficial in the above-mentioned case. It allows to utilise the most of the advantages presented by crystallization as a low-temperature technique, and minimise energy consumption and the degree of side fraction formation, as is the case with high-temperature techniques, e.g. distillation. Melt crystallization is based on the recovery of a pure crystalline product from a molten concentrate and separating it from the mother liquid containing impurities (Melt Crystallization Technology, GF Arkenbout, Technomic, 1995, ISBN 1-56676-181-6*).*

## Claims

**1.** A method of selective paradichlorobenzene preparation with improved catalytic system recovery, comprising a chlorination of benzene and/or monochlorobenzene with molecular chlorine in the presence of a catalytic system comprising antimony trichloride as a catalyst and N-chlorocarbonylphenothiazine as an organic cocatalyst, **characterised in that**

(a) a reaction mixture composed of fresh raw material input and streams obtained as a result of recycling a fraction of unreacted raw materials together with recovery of the catalytic system is subjected to chlorination, and then
(b) the catalytic system is recovered by separating a fresh post-reaction mixture obtained in (a) in the following stages:

(I) distilling off light fractions under reduced pressure from the fresh post-reaction mixture, including unreacted benzene and/or monochlorobenzene;
(II) crystallization of the desired paradichlorobenzene with the collection of paradichlorobenzene as a pure product stream; after which
(III) the obtained mother liquor containing the catalytic system and the fraction of unreacted benzene and/or monochlorobenzene raw materials from (I) is recycled.

**2.** The method according to claim 1, **characterised in that** a reaction mixture is provided in step (a) of this method, composed of a uniform mixture of fresh raw material input containing benzene and/or monochlorobenzene and a fraction of unreacted raw materials together with the recovered catalytic system, delivered homogeneously directly to the reactor.

**3.** The method according to any one of claims 1 or 2, **characterised in that** the catalytic system is used in step (a) of this method, in the amount of: 0.1 wt.% antimony trichloride relative to the weight of the reaction mixture.

**4.** The method according to any one of claims 1-3, **characterised in that** the reaction mixture containing benzene and/or monochlorobenzene in a volume ratio of 0-75 vol% of benzene and 25-100 vol% of monochlorobenzene, preferably 0-25 vol% of benzene and 75-100 vol% of monochlorobenzene is used in step (a) of this method.

**5.** The method according to any one of claims 1-4, **characterised in that** the chlorination process is carried out in the temperature range from 50 to 70°C, preferably at 60°C, in step (a) of this method.

**6.** The method according to any one of claims 1-5, **characterised in that** N-chlorocarbonylphenothiazine is used with antimony trichloride at a molar ratio of 0.5-1.5:1, preferably at a molar ratio of 1:1, in step (a) of this method.

**7.** The method according to any one of claims 1-6, **characterised in that** the catalytic system is supplemented depending on the amount of recovered antimony trichloride and N-chlorocarbonylphenothiazine in step (a) of this method.

8. The method according to any one of claims 1-7, **characterised in that** mother liquor from (III) is used as a recovery to be recycled in step (a) of this method, preferably containing the catalytic system in amounts above 55% of the catalyst and above 60% of the cocatalyst, in relation to the input amount.

9. The method according to any one of claims 1-8, **characterised in that** the reaction mixture is supplemented with a fresh portion of raw materials consisting of benzene and/or monochlorobenzene, and optionally a fresh portion of the catalytic system for recovery from step (b), is used in step (a) of this method.

10. The method according to any one of claims 1-9, **characterised in that** chlorine gas is introduced into the reactor at a pressure of 0.1-5 bar, preferably 0.3-1 bar, and most preferably 0.4-0 .5 bar in step (a) of this method.

11. The method according to any one of claims 1-10, **characterised in that** the chlorination process is carried out for 4-15 h, preferably 6-10 h in step (a) of this method.

12. The method according to any one of claims 1-11, **characterised in that** a three-stage separation process is used in step (b) of the method, including distillation under reduced pressure, crystallization, preferably melt crystallization, and filtration, while maintaining the highly selective catalytic capacity of the above-mentioned catalytic system.

13. The method according to any of claims 1-12, **characterised in that** the distillation step (I) for benzene is carried out under vacuum conditions of 0-1000 mbar, preferably 120-180 mbar, and most preferably 140-145 mbar - which results in a boiling point of 45-50°C and/or it is carried out under vacuum conditions of 0-1000 mbar; preferably 60-100 mbar, and most preferably 80-90 mbar for monochlorobenzene - which yields a boiling point of 55-60°C.

14. The method according to any of claims 1-13, **characterised in that** step (II) of this method involves the crystallization of the desired paradichlorobenzene with the paradichlorobenzene collection as a stream of pure product in crystalline form or in melt form.

15. The method according to any of claims 1-14, **characterised in that** step (II) of this method involves crystallization, which involves slowly lowering the temperature at a rate of 0.1-5°C/min, preferably 0.1-1°C/min; and most preferably 0.2-0.3°C/min, while mixing slowly in the range of 20-100 rpm, and most preferably 50-60 rpm.

16. The method according to any of claims 1-15, **characterised in that** the mother liquid obtained by filtration is recycled for repeat use in (a) in step (III) of this method.

17. The method according to any of claims 1-16, **characterised in that** it is carried out in a batch reactor, a continuous reactor or in a tubular reactor, a microchannel reactor, an overflow reactor with a mechanical stirrer, a "bubble column" reactor, a "loop-reactor" or in any other types of reactors.

18. The method according to any one of claims 1-17, **characterised in that** instead of antimony trichloride, another Lewis acid may be used in step (a) of this method, including iron chloride, aluminium chloride, magnesium chloride and zinc chloride, preferably iron chloride or aluminium chloride.

Fig. 1

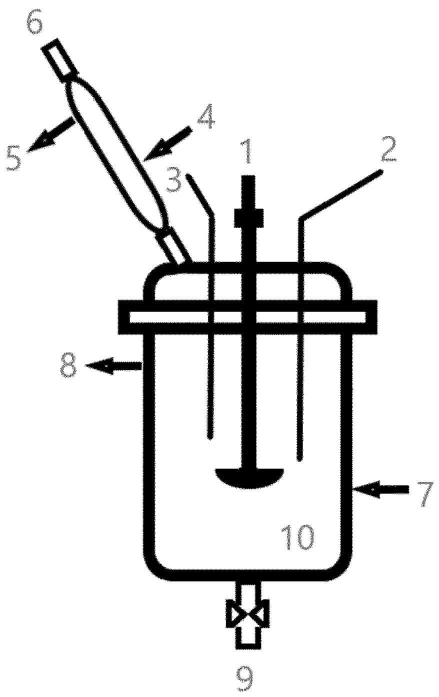


Fig. 2

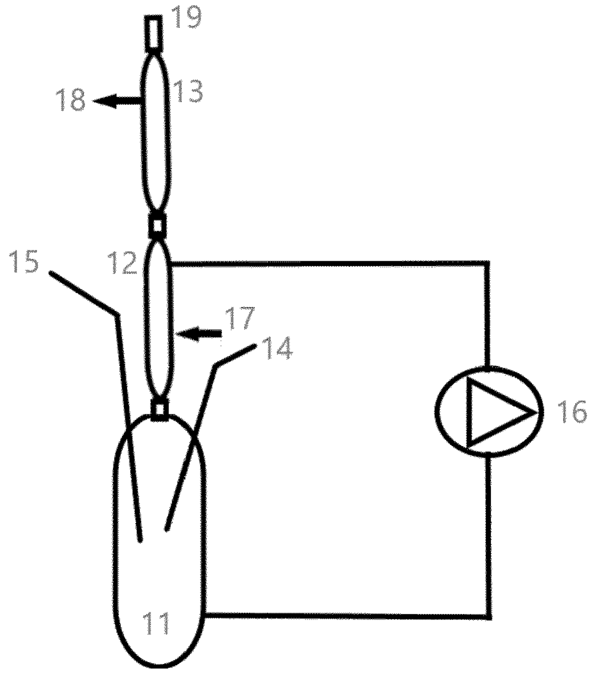

Europäisches Patentamt
European Patent Office
Office européen des brevets

# EUROPEAN SEARCH REPORT

Application Number
EP 23 20 5718

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 351 724 B1 (TOSOH CORP [JP]) 26 November 2014 (2014-11-26) * examples 3-10, 12 * ----- | 1-18 | INV. C07C17/02 C07C17/04 C07C25/08 |
| X | CN 103 819 307 A (HULUDAO JINHUA CHEMICAL INDUSTRY ENGINEERING DESIGN CO LTD) 28 May 2014 (2014-05-28) * examples 1, 2 * ----- | 1-18 | |
| | | | TECHNICAL FIELDS SEARCHED (IPC) |
| | | | C07C |

The present search report has been drawn up for all claims

1

| Place of search | Date of completion of the search | Examiner |
|---|---|---|
| The Hague | 18 March 2024 | Tabanella, Stefania |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 20 5718

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2024

| Patent document cited in search report | Publication date | Patent family member(s) | Publication date |
|---|---|---|---|
| EP 2351724 B1 | 26-11-2014 | CN 102197010 A | 21-09-2011 |
| | | EP 2351724 A1 | 03-08-2011 |
| | | JP 5493700 B2 | 14-05-2014 |
| | | JP 2010120934 A | 03-06-2010 |
| | | US 2011207976 A1 | 25-08-2011 |
| | | WO 2010047392 A1 | 29-04-2010 |
| CN 103819307 A | 28-05-2014 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2328690 A
- US 4511552 A
- GB 1337916 A
- AU 251182 B
- GB 971044 A
- US 3354129 A
- WO 199008181 A
- US 3029296 A
- US 2111866 A
- US 3636171 A
- US 4017551 A
- WO 2007017900 A2
- US 3226447 A
- EP 0126669 B1
- EP 23551724 B1
- JP 5375024 B
- JP 381832182 B
- EP 0474074 A1
- EP 0153589 B1
- EP 0505874 A1
- JP 2002114719 A
- EP 0313990 A2
- KR 100188339 B1
- EP 0195514 B1
- EP 0154236 B1
- CN 1023618418
- JP 565886582 B
- WO 2010122925 A1
- CN 109574789 A
- EP 0118851 A1
- JP 6287536 A
- EP 0231133 B1
- EP 0171265 B1
- CN 103819307 A
- CN 112723987 A
- US 4300004 A
- EP 045172 A
- KR 100352539 B1
- DE 3617137 A
- DE 2855940 B1
- US 4762955 A
- CN 102976888 A
- EP 2351724 B1
- CN 102361841 B


### Non-patent literature cited in the description


- Ullmann's Encyclopedia of Industrial Chemistry,. vol. A6, 333
- *CHEMICAL ABSTRACTS,* 10025-91-9
- *CHEMICAL ABSTRACTS,* 18956-87-1
- **GF ARKENBOUT.** Technomic. *Melt Crystallization Technology,* 1995, ISBN 1-56676-181-6